(19) Europäisches Patentamt
European Patent Office
Office européen des brevets

(11) **EP 3 167 284 B1**

(12) **EUROPEAN PATENT SPECIFICATION**

(45) Date of publication and mention
of the grant of the patent:
**02.03.2022 Bulletin 2022/09**

(21) Application number: **15804999.9**

(22) Date of filing: **05.11.2015**

(51) International Patent Classification (IPC):
**G01N 31/12** (2006.01)     **E21B 49/00** (2006.01)
**G01N 33/24** (2006.01)

(52) Cooperative Patent Classification (CPC):
**G01N 33/241; G01N 1/286; G01N 1/4022;**
**G01N 31/12;** G01N 2001/4033

(86) International application number:
**PCT/US2015/059147**

(87) International publication number:
**WO 2016/186689 (24.11.2016 Gazette 2016/47)**

(54) **PYROLYSIS TO DETERMINE HYDROCARBON EXPULSION EFFICIENCY OF HYDROCARBON SOURCE ROCK**

PYROLYSE ZUR BESTIMMUNG DER KOHLENWASSERSTOFFAUSSTOSSEFFIZIENZ VON KOHLENWASSERSTOFFMUTTERGESTEIN

PYROLYSE POUR DÉTERMINER UN RENDEMENT D'EXPULSION D'HYDROCARBURES DE ROCHE MÈRE D'HYDROCARBURES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2015 US 201514717825**

(43) Date of publication of application:
**17.05.2017 Bulletin 2017/20**

(73) Proprietor: **Saudi Arabian Oil Company Dhahran 31311 (SA)**

(72) Inventor: **INAN, Sedat Dhahran 31311 (SA)**

(74) Representative: **Fish & Richardson P.C. Highlight Business Towers Mies-van-der-Rohe-Straße 8 80807 München (DE)**

(56) References cited:
**GB-A- 2 161 269     US-A- 5 201 219**

• **CHRIS CORNFORD: "Risking Petroleum Systems", AAAPG 2000. PROCEEDINGS OF THE FIFTH INTERNATIONAL CONFERENCE AND EXHIBITION ON PETROLEUM GEOCHEMISTRY AND EXPLORATION IN THE AFRO-ASIAN REGION, 1 January 2000 (2000-01-01), XP055244998,**
• **ESEME ET AL: "Experimental investigation of the compositional variation of petroleum during primary migration", ORGANIC GEOCHEMISTRY, PERGAMON, AMSTERDAM, NL, vol. 38, no. 8, 29 July 2007 (2007-07-29), pages 1373-1397, XP022176336, ISSN: 0146-6380, DOI: 10.1016/J.ORGGEOCHEM.2007.03.003**

**Description**

CLAIM OF PRIORITY

This application claims priority to U.S. Patent Application No. 14/717,825 filed on May 20, 2015.

TECHNICAL FIELD

This specification relates to analyzing rock in which hydrocarbon is generated and trapped.

BACKGROUND

Hydrocarbon exploration techniques sometimes involve generating computer-generated geological models and calibrating such models using experimental data. For example, the experimental data can be provided as inputs to the geological models. The experimental data can be obtained from laboratory experiments performed on hydrocarbon source rock, that is, rock in which hydrocarbons are generated. The accuracy of the predictions of computer-generated models can depend on the quality of the calibration of the models using measured experimental data, which, in turn, can depend on the conditions under which the laboratory experiments are performed. Said differently, the quality of the experimental data can be high if the conditions under which the laboratory experiments are performed are substantially similar to the conditions experienced by the rock in the subsurface from which the hydrocarbons are produced. One input to the geological model can include a characteristic of the hydrocarbon rock to expel trapped hydrocarbons. Pyrolysis is one technique to study the characteristic of hydrocarbon source rock to expel or trap hydrocarbons.

The document entitled "Risking Petroleum Systems" by Chris Cornford (Proc. 5th Int. Con. Exhib. Petrol. Geochem. Explor. Afro-Asian Region, 1 January 2000 (2000-01-01)) describes petroleum systems and inputs into exploration and production decision making.

The document entitled "Experimental investigation of the compositional variation of petroleum during primary migration" by Eseme et al, Org. Geochem 38(8) p. 1373-1397 (2007) describes anhydrous non-isothermal heating experiments were conducted under controlled compressive stress on cylindrical plugs of six oil shales from Permian through Miocene age.

GB 2161269 A describes a method of determining hydrocarbons present in a sample in which the sample is pyrolyzed, the off stream split into two with one stream analyzed for total hydrocarbons present and the other stream passed through traps to separate the oil range hydrocarbons from the gas range hydrocarbons.

SUMMARY

This specification describes pyrolysis techniques to analyze the characteristic of a hydrocarbon source rock to expel trapped hydrocarbons. In particular, for example, this specification describes pyrolysis to determine hydrocarbon expulsion efficiency of hydrocarbon source rock.

One aspect of the invention is a method according to independent claim 1, whereby an open system pyrolysis is performed on a hydrocarbon source rock sample obtained from a natural system. The hydrocarbon source rock sample includes hydrocarbon source rocks having an equivalent spherical diameter of substantially at least one centimeter. Hydrocarbons released by the hydrocarbon source rock sample in response to the open system pyrolysis are recovered. After crushing the pyrolyzed hydrocarbon source rock sample a thermo-vaporization is performed on the pyrolyzed hydrocarbon source rock sample on which the open system pyrolysis was performed. Hydrocarbons released by the pyrolyzed hydrocarbon source rock sample in response to the thermo-vaporization are recovered. A hydrocarbon expulsion efficiency of hydrocarbou source rock in the natural system is determined based on the recovered hydrocarbons released by the hydrocarbon source rock sample in response to the open system pyrolysis and based on the recovered hydrocarbons released by the pyrolyzed hydrocarbon source rock sample in response to thermo-vaporization.

This, and other aspects, can include one or more of the following features. The natural system can include a subsurface system including a hydrocarbon reservoir. The pyrolyzed hydrocarbon source rock is crushed prior to performing the thermo-vaporization. To determine the hydrocarbon expulsion efficiency, a first quantity of hydrocarbons released in response to performing the open system pyrolysis can be determined. A second quantity of hydrocarbons released in response to performing the thermo-vaporization can be determined. To determine the hydrocarbon expulsion efficiency, a ratio of the first quantity of hydrocarbons to a sum of the first quantity of hydrocarbons and the second quantity of hydrocarbons can be determined. The open system pyrolysis of the hydrocarbon source rock sample can be performed in a pyrolysis chamber. To perform the open system pyrolysis of the hydrocarbon source rock, a hydrocarbons transport can be flowed through the pyrolysis chamber while performing the open system pyrolysis. The hydrocarbons transport can be configured to carry the hydrocarbons released by the hydrocarbon source rock sample. The hydrocarbons transport can include an inert gas. A volume of the pyrolysis chamber can be at least one liter. The thermo-vaporization on the pyrolyzed hydrocarbon source rock sample can be performed in a thermo-vaporization chamber. To perform the thermo-vaporization on the pyrolyzed hydrocarbon source rock sample, an inert hydrocarbons transport can be flowed through the thermo-vaporization chamber while performing the thermo-vaporization. The inert hydrocarbons transport can be configured to carry the hydrocarbons released from the pyrolyzed hydrocarbon source rock sample and which was retained within the sample during open system pyrolysis. The pyrolyzed hydrocarbon source rock sample is crushed in the thermo-vaporization chamber prior to performing the thermo-vaporization. The open system pyrolysis of the hydrocarbon source rock sample, crushing the pyrolyzed hydrocarbon source rock sample and thermo-vaporization of the pyrolyzed hydrocarbon source rock sample can be performed in the thermo-vaporization chamber. A quantity of the hydrocarbon source rock sample used in the open system pyrolysis can be substantially at least 100 g. To recover hydrocarbons released by the hydrocarbon source rock sample in response to the open system pyrolysis, the hydrocarbons released by the hydrocarbon source rock sample can be flowed to a cold trap including a fused silica column submerged in liquid nitrogen. The cold trap can be maintained a temperature of substantially -100 °C until an end of the open system pyrolysis. The cold trap can trap at least a portion of the hydrocarbons released by the hydrocarbon source rock sample. The cold trap can be heated after the end of the open system pyrolysis to release the trapped portion of the hydrocarbons. The recovered hydrocarbons released by the hydrocarbon source rock sample in response to the open system pyrolysis can be flowed to instrumentation configured to analyze composition of the recovered hydrocarbons. The instrumentation can include a gas chromatograph or a gas chromatography mass spectrometer. The recovered hydrocarbons released by the hydrocarbon source rock sample can be split into multiple hydrocarbon gas streams. A first stream can be flowed to a first instrumentation configured to analyze a composition of the first stream. A second stream can be flowed to a second instrumentation configured to analyze a composition of the second stream. A third stream can be released to atmosphere. To perform the open system pyrolysis of the hydrocarbon source rock sample, the hydrocarbon source rock sample can be heated to one or more of multiple temperatures up to 650 °C. To heat the hydrocarbon source rock sample to one or more of multiple temperatures, the hydrocarbon source rock sample can be heated at different heating rates. A heating rate is a time rate of temperature increase from a lower temperature to a higher temperature. To perform the thermo-vaporization on the pyrolyzed hydrocarbon source rock sample, the pyrolyzed hydrocarbon source rock sample can be heated at constant temperature of substantially 300 °C. The hydrocarbon expulsion efficiency can be provided as an input to a computer-generated geological model to study hydrocarbon expulsion efficiency from a hydrocarbon source rock through a geological history of the hydrocarbon source rock.

Another aspect of the invention is a system according to independent claim 16, whereby an open system pyrolysis apparatus configured to pyrolyze fragments of a hydrocarbon source rock sample. The fragments have an equivalent spherical diameter of substantially at least one centimeter. The system includes a crushing and thermo-vaporization apparatus configured to crush and simultaneously thermally vaporize released hydrocarbons from previously pyrolyzed hydrocarbon source rock sample. The system includes a hydrocarbon transport apparatus configured to recover hydrocarbons released by the hydrocarbon rock sample in response to the open system pyrolysis and hydrocarbon released by the previously pyrolyzed hydrocarbon source rock sample in response to the crushing and thermo-vaporization. The system includes instrumentation configured to receive the recovered hydrocarbons released by the hydrocarbon source rock sample in response to the open system pyrolysis and the hydrocarbon released by the pyrolyzed hydrocarbon source rock sample in response to the crushing and thermo-vaporization.

The details of one or more implementations of the subject matter described in this specification are set forth in the accompanying drawings and the description below. Other features and advantages of the subject matter will become apparent from the description, the drawings, and the claims.

BRIEF DESCRIPTION OF THE DRAWINGS

Figs. 1A and 1B are schematic diagrams of an example of a system for artificially maturing a hydrocarbon source rock sample.
Fig. 2 is a flowchart of an example of a process for determining a hydrocarbon expulsion efficiency of hydrocarbon source rock.
Fig. 3 is a plot of an example of a pyrolysis temperature intervals (steps).
Fig. 4 is a plot of an example of a thermo-vaporization in a sample crusher chamber at constant temperature.
Fig. 5A is a plot of an example of maturity increase based on pyrolysis temperature steps.
Fig. 5B is an example of hydrocarbon distributions recovered after pyrolysis and/or crushing-thermo-vaporization stage from a hydrocarbon source rock sample.

Like reference numbers and designations in the various drawings indicate like elements.

DETAILED DESCRIPTION

This specification describes methods and systems associated with the exploration for petroleum. In particular, this specification describes pyrolysis methods and systems to determine expulsion of hydrocarbons from hydrocarbon source rock. The pyrolysis methods and systems described here can be implemented in a laboratory and, in some implementations, the results obtained can be provided as inputs to computer-generated geological models used to study hydrocarbon generation and expulsion from hydrocarbon source rocks.

The expulsion of hydrocarbons from hydrocarbon source rock into reservoir rock, from which the hydrocarbon can be recovered, is affected by the release of generated hydrocarbons from kerogen and movement (that is, migration) of these hydrocarbons within the source rock. The release of liquid hydrocarbons from kerogen is controlled by the absorption or adsorption (or both) of the liquid hydrocarbons within or on the surface (or both) of the kerogen and diffusion of the hydrocarbons through kerogen. Thus, the efficiency of expulsion is controlled by the amount and oil-proneness (or hydrogen-richness) of kerogen.

The migration of oil within the source rock is affected by factors including, for example, an amount and type of organic matter, bitumen (oil) saturation threshold, effective migration paths in the hydrocarbon rock, pressure build-up and micro-fracturing, gas availability for the movement of oil in the gaseous phase, combinations of them, or other factors. These factors vary with the primary sedimentological conditions in the depositional environment of the source rock and secondary diagenetic processes during evolution of sedimentary basins. In other words, the mechanism of hydrocarbon migration will vary according to the litho-facies of the hydrocarbon source rock.

Laboratory pyrolysis is used to artificially mature kerogen and study the processes of hydrocarbon generation within and expulsion from the source rock. Laboratory pyrolysis experiments can be conducted in closed or open systems, by isothermal or non-isothermal heating of finely ground (for example, in powder form) hydrocarbon source rock samples, for example, in hydrous or anhydrous tubes. Open system pyrolysis can be conducted using, for example, a few milligrams of ground rock having a size of less than approximately 250 micrometer. In open system pyrolysis, the yields (that is, the hydrocarbons expelled by pyrolysis) can be removed from the system (sometimes immediately after pyrolysis) by hydrocarbons transport. Closed system pyrolysis, for example, closed anhydrous systems, can be implemented in a micro-scale sealed vessel or implemented as gold-bag pyrolysis systems utilizing small amount of hydrocarbon source rock or isolated kerogen. In closed system pyrolysis, the generated hydrocarbons can remain in the system and be exposed to further heating until the end of pyrolysis, for example, until secondary cracking occurs. Hydrous pyrolysis involves heating of kerogen in presence of excess water. Such pyrolysis is conducted in sealed reactors and leads to secondary reactions and cracking of generated hydrocarbons within the pyrolysis chamber. Thus, different types of pyrolysis can be implemented to artificially mature kerogen to study the processes of hydrocarbon generation and migration. Such artificial kerogen maturation, while a useful tool, does not always accurately represent maturation of sedimentary organic matter in natural systems, that is, in the subsurface.

This disclosure describes an artificial kerogen maturation technique that better approximates natural maturation of kerogen relative to the artificial kerogen maturation techniques described earlier. In some implementations, the artificial kerogen maturation technique described here can be implemented as a restricted system pyrolysis. The pyrolysis system is restricted in that, to be expelled due to pyrolysis, the hydrocarbons in the hydrocarbon source rock need to overcome a physical barrier before the hydrocarbons can reach a space from which the expelled hydrocarbons can be swept away by hydrocarbons transport. In other words, the restricted pyrolysis technique described in this specification studies the ability of the hydrocarbons in the rock sample to traverse through a physical barrier that is similar to the physical barrier that hydrocarbon generated during natural maturation would experience in the subsurface (for example, subsurface hydrocarbon reservoirs).

The techniques described here can be implemented to predict hydrocarbon accumulations or to predict hydrocarbons retained in hydrocarbon source rocks (or both). The techniques can also be implemented to determine an expulsion efficiency of hydrocarbons from hydrocarbon source rock samples, and to input such efficiency in computer-generated geological models, for example, models that can predict hydrocarbon accumulations. The expulsion efficiency obtained by implementing the techniques described here can increase a confidence in the computer-generated geographic models implemented in hydrocarbon exploration or recovery or both. For example, the expulsion efficiency can be used to calibrate petroleum system and basin modeling simulation which can lead to better understanding and determination of expulsion of hydrocarbons from source rock. The calibrate systems and simulations can be implemented to successfully discover hydrocarbon reservoirs, to better predict retained hydrocarbons in such reservoirs, or to identify sweet spots in unconventional resource estimation (or combinations of them). The time and effort in performing such processes can thereby be reduced to increase efficiency.

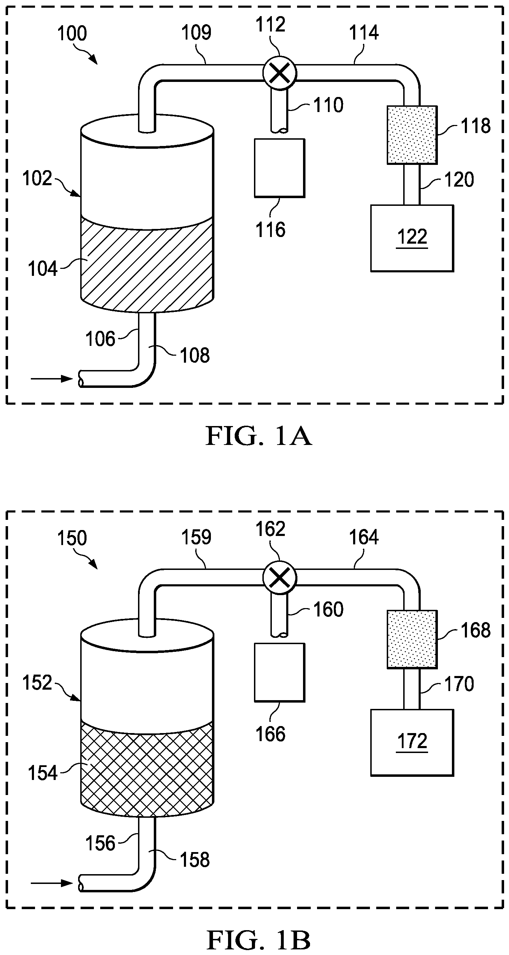

Figs. 1A and 1B are schematic diagrams of an example of a system for artificially maturing a hydrocarbon source rock sample. Fig. 1A is a schematic diagram of an example of an open system pyrolysis apparatus 100. The apparatus 100 includes a pyrolysis chamber 102 in which a rock sample 104 is placed. The rock sample 104 includes fragments of the rock sample having an equivalent spherical diameter of substantially at least one centimeter (for example, ranging between 0.9 cm and several centimeters). The equivalent spherical diameter of the fragments of the rock sample 104 is the diameter of a sphere having an equivalent volume as the rock sample 104. In some implementations, the hydrocarbon source rock sample 104 can include multiple pieces of hydrocarbon rock at least some of which have the equivalent spherical diameter of substantially at least one centimeter. Some fragments can have an equivalent spherical diameter different from (that is, lesser than or greater than) at least one centimeter. In general, the hydrocarbon source rock sample 104 can have a size that is substantially similar to the size of equivalent hydrocarbon rock that is found in the subsurface from which the rock sample 104 is obtained.

The hydrocarbon source rock sample 104 can be obtained from a hydrocarbon source rock to be studied. The grain size of hydrocarbon source rock in the subsurface affects a distance through which hydrocarbons generated and trapped in the rock need to traverse before being released during natural kerogen maturation. The hydrocarbon source rock sample 104 is selected to have a grain size that is substantially similar to a grain size of hydrocarbon rock in the subsurface. In this manner, the experimental conditions of the artificial kerogen maturation can be made closer to the conditions of natural kerogen maturation. That is, in such conditions, the hydrocarbon in the rock sample 104 will need to traverse a distance that is closer to the distance through which the hydrocarbons trapped in the rock need to traverse before being released during natural kerogen maturation. The hydrocarbon expulsion efficiency determined for the hydrocarbon source rock sample 104 following the artificial kerogen maturation will, consequently, be a more accurate prediction of the hydrocarbon expulsion efficiency of the hydrocarbon source rock in the subsurface following natural kerogen maturation.

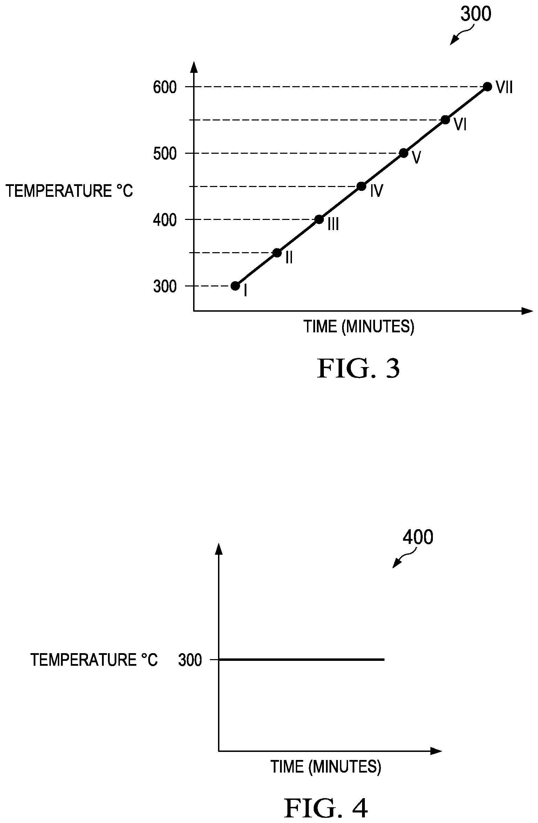

The pyrolysis chamber 102 is larger than conventional pyrolysis chambers used in the pyrolysis of hydrocarbon source rock samples. For example, the pyrolysis chamber 102 can have a volume of substantially 1 liter (for example, ranging between 0.8 liters and few liters), and can be configured to hold several hundred grams (for example, up to nearly 800 g) of hydrocarbon source rock sample. The pyrolysis chamber 102 can further be configured to heat the hydrocarbon source rock sample 104 to temperatures as high as 650 °C. In particular, the pyrolysis chamber 102 can be configured to heat the hydrocarbon source rock sample at different temperature gradients and different temperature steps as described below with reference to Fig. 3.

In some implementations, the hydrocarbon source rock sample 104 can be of a larger size compared to hydrocarbon source rock samples used in conventional pyrolysis chambers. For example, the hydrocarbon source rock sample 104 can have grain sizes in the centimeter range. In addition, a quantity of the hydrocarbon source rock sample 104 used in the pyrolysis chamber 102 during open system pyrolysis can range between about 100 g and about 800 g. In some implementations, the hydrocarbon source rock sample 104 can additionally be characterized in terms of the Total Organic Carbon (TOC) content as weight percent (wt %) of the source rock and determination of kerogen type using conventional organic geochemical techniques. The TOC content can be determined by LECO TOC analyzer where the organic carbon is measured after removal of carbonate carbon. The TOC content can also be determined by Source Rock Analyzers which have pyrolysis and oxidation ovens.

In some implementations, a hydrocarbons transport (carrier) apparatus can be connected to the open system pyrolysis apparatus 100. For example, the hydrocarbons transport apparatus can include tubing 106 connected to a bottom side of the pyrolysis chamber 102. As described below, hydrocarbons transport 108 can be flowed through the pyrolysis chamber 102. The hydrocarbons transport apparatus can also include tubing 109 connected to the pyrolysis chamber 102 (for example, on an upper side or other side) through which the hydrocarbons transport 108 can transport the hydrocarbons released from the hydrocarbon source rock sample 104 during or soon after pyrolysis (or at both times). The hydrocarbons transport apparatus can include one or more valves (for example, valve 112) to control the flow of the recovered hydrocarbons. In some implementations, the hydrocarbons recovery apparatus can include additional tubings (for example, tubing 110, tubing 114, tubing 120) to transport all or portions of the recovered hydrocarbons to one or more cold traps (for example, cold trap 116, cold trap 118, or other cold traps). This apparatus can also be configured to flow all or portions of the recovered hydrocarbons to instrumentation 122 (for example, a gas chromatograph or other instrumentation) that is configured to analyze the hydrocarbons, for example, to determine a compositional analysis of the recovered hydrocarbons.

Fig. 1B is a schematic diagram of an example of a sample-crushing and thermo-vaporization apparatus 150. The apparatus 100 includes a sample-crusher and thermo-vaporization chamber 152 in which the previously pyrolyzed hydrocarbon source rock sample 154 is placed. In some implementations, this chamber which includes a sample crusher apparatus insidethermo-vaporization152 can be separate and distinct from the pyrolysis chamber 102, and can be configured to heat the pyrolyzed hydrocarbon source rock sample 154 to temperatures as high as 300°C. In some implementations, the sample crusher-thermo-vaporization chamber 152 and the pyrolysis chamber 102 can be the same. That is, after completing the open system pyrolysis, the pyrolysis chamber 102 shown in Fig. 1A can be re-used to perform sample crushing and thermo-vaporization on the previously pyrolyzed hydrocarbon source rock sample.

In some implementations, a hydrocarbon transport apparatus can be connected to the sample crushing and thermo-vaporization apparatus 150. For example, the hydrocarbon transport apparatus can include tubing 156 connected to a bottom side of the sample crushing and thermo-vaporization chamber 152. As described below, hydrocarbons transport 158 can be flowed through the thermo-vaporization chamber 152. The hydrocarbons recovery apparatus can also include tubing 159 connected to the thermo-vaporization chamber 152 (for example, on an upper side or other side) through which the hydrocarbons transport 158 can transport the hydrocarbons released during crushing and thermo-vaporization of the previously pyrolyzed hydrocarbon source rock sample 154thermo-vaporization. The hydrocarbon transport apparatus can include one or more valves (for example, valve 162) to control the flow of the recovered hydrocarbons. In some implementations, the apparatus can include additional tubings (for example, tubing 160, tubing 164, tubing 170) to transport all or portions of the recovered hydrocarbons to one or more cold traps (for example, cold trap 176, cold trap 178, or other cold traps). The apparatus can also be configured to flow all or portions of the recovered hydrocarbons to instrumentation 172 (for example, a chromatograph or other instrumentation) that is configured to analyze the hydrocarbons, for example, to determine chemical composition.

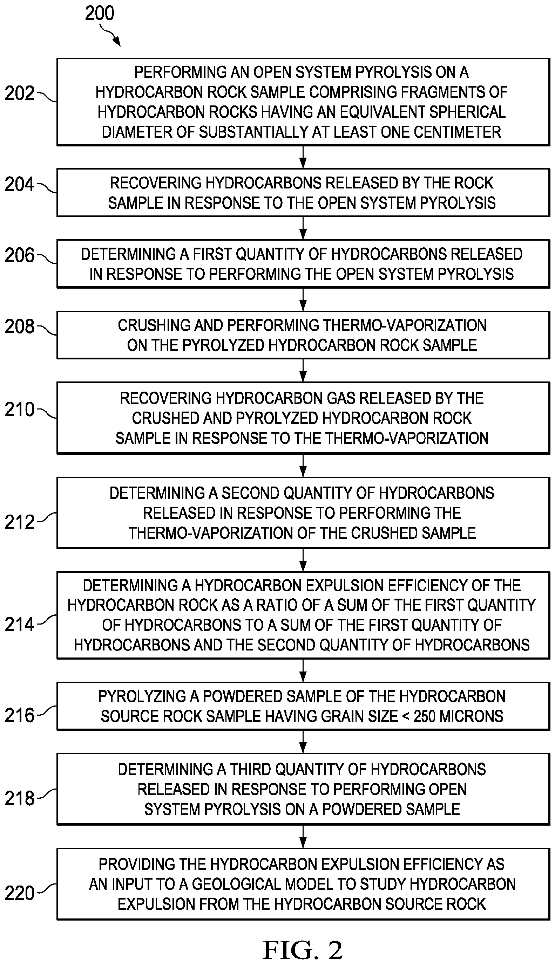

Fig. 2 is a flowchart of an example of a process 200 for determining a hydrocarbon expulsion efficiency of hydrocarbon source rock. The process 200 can be implemented, for example, using the systems described above with reference to Figs. 1A and 1B. At 202, an open system pyrolysis can be performed on fragments of a hydrocarbon source rock sample obtained from subsurface. The hydrocarbon source rock sample can include fragments having an equivalent spherical diameter of substantially at least one centimeter. A quantity of the hydrocarbon source rock sample 104 used in open system pyrolysis can be a few hundred grams (for example, substantially 100 g). During open system pyrolysis, the hydrocarbon source rock sample 104 is placed in the pyrolysis chamber 102 and heated non-isothermally. In some implementations, the temperature of the pyrolysis chamber 102 can be increased at different heating rates. Fig. 3 is a plot 300 of an example of a pyrolysis temperature steps according to which the hydrocarbon source rock sample 104 can be pyrolyzed in the open system pyrolysis apparatus 100. For example, the temperature of the pyrolysis chamber 102 can be increased at a heating rate of substantially about 25 °C per minute (or lower or higher heating rates).

In some implementations, the pyrolysis can be conducted in multiple heating heating rates, for example, eight temperature stages (steps). The heating rate can be the same or different in each temperature stage. The increase in the heating rate between any consecutive temperature steps can be the same or different. For example, the increase in the heating gradient between consecutive temperature steps can remain 5 °C per minute for all temperature steps.

At 204, hydrocarbons released by the hydrocarbon source rock sample in response to the open system pyrolysis can be recovered. In some implementations, the pyrolysis chamber 102 can be continuously flushed with the inert hydrocarbons transport 108 (for example, helium or other inert hydrocarbons transport) using the hydrocarbon transport apparatus. For example, the hydrocarbons transport 108 can be flowed into the pyrolysis chamber 102 through tubing 106 and out of the pyrolysis chamber 102 with the expelled hydrocarbons through tubing 110. As the hydrocarbons in the hydrocarbon source rock sample 104 are volatile in gaseous form at high temperatures, the hydrocarbons transport 108 can carry the expelled hydrocarbons out of the pyrolysis chamber 102. In some implementations, hydrocarbons expelled from the hydrocarbon source rock sample 104 can be flushed out of the pyrolysis chamber 102 after each temperature stage (e.g. 300-350 °C step). During a pyrolysis both tubings 106 and 110 are continuously open to flush the expelled hydrocarbons.

The combination of the hydrocarbons and the hydrocarbons transport 108 can be flowed to one or more cold traps (for example, the cold trap 116 or the cold trap 118 or both). In some implementations, each cold trap can be a fused silica column submerged in a container filled with liquid nitrogen or other low temperature (for example, as low as -100 °C). Each cold trap can trap at least a portion of the hydrocarbons released by the hydrocarbon source rock sample either after open system pyrolysis or after thermo-vaporization. For example, the cold trap may not trap methane gas. At the end of any pyrolysis temperature step (for example, each temperature step, any one of the temperature steps, or the last temperature step), the cold trap can be heated first to a temperature of about 60 °C and then to an elevated temperature of about 300 °C to purge the gaseous and liquid pyrolysis products, respectively. In this manner, the hydrocarbons trapped by the cold trap 118 can be released.

In some implementations, the hydrocarbon transport apparatus can be implemented to split the hydrocarbons carried by the hydrocarbons transport and flowing out of the pyrolysis chamber 102 into multiple streams. For example, the valve 112 can split the hydrocarbons transport flowing from the pyrolysis chamber 102 into a first gas stream and a second gas stream. The first gas stream can be directed to a removable cold trap 118 and the second portion can be directed to the cold trap 116. The first gas stream flowed through the removable cold trap 116 can then be flowed to another instrumentation (not shown) to determine a composition of the hydrocarbons. In some implementations, the first gas stream can be purged, for example, released to atmosphere in a controlled vacuum hood. In some implementations, the gas stream exiting the pyrolysis chamber 102 can be split into multiple streams (that is, more than two streams). One of the streams can be flowed to the cold trap 118 and then to the instrumentation 122. Another stream can be flowed to the removable cold trap 116 and then to another instrumentation. A third stream can be purged, and so on. The splitting of the gas stream exiting the pyrolysis chamber 102 into multiple gas streams and the flow of the gas streams can be controlled, for example, using the valve 112.

At 206, a first quantity of hydrocarbons released in response to performing the open system pyrolysis can be determined. In some implementations, the hydrocarbons carried by the hydrocarbons transport 108 out of the pyrolysis chamber 102 can be flowed to instrumentation 122 that can analyze composition of the expelled hydrocarbons. For example, the instrumentation 114 can include a gas chromatograph (GC) or gas chromatography mass spectrometer (GC-MS). The instrumentation 122 can be implemented to characterize the hydrocarbons expelled from the rock sample 104. In some implementations, the instrumentation 122 can be implemented to quantify, for example, in micrograms of hydrocarbons per gram of hydrocarbon source rock sample.

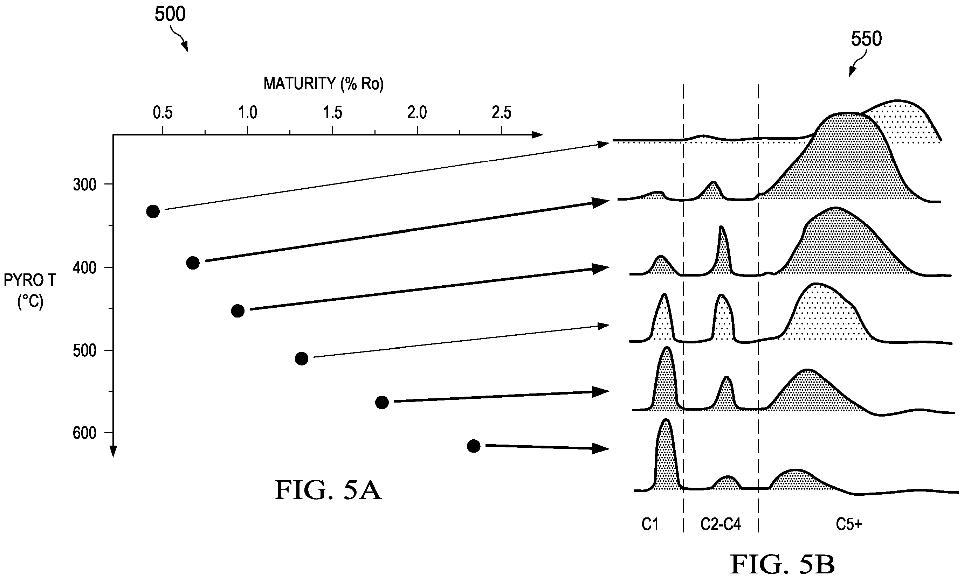

Fig. 5A is a plot of an example of pyrolysis temperature to maturity increase of hydrocarbon source rock sample. Fig. 5B is an example of hydrocarbon class (methane, wet gas, light oil, heavy oil, etc.) distribution in hydrocarbons recovered from a hydrocarbon source rock sample. The two figures show the maturity of the hydrocarbon source rock sample 102 reached at the end of each pyrolysis stage. The two figures also show with expected variations of hydrocarbon class distribution ($C_1$ (methane), $C_2$-$C_4$ (ethane, propane and butane), $C_5$+ (pentane and heavier hydrocarbons)) with increasing maturity. That is, as the pyrolysis temperature stage increases, more maturity is attained and the lighter hydrocarbon class (for example, $C_1$-$C_4$) dominates.

At 208, a thermo-vaporization can be performed on the pyrolyzed hydrocarbon source rock sample on which the open system pyrolysis was performed. In some implementations, after the open system pyrolysis, the hydrocarbon source rock sample is removed from the pyrolysis chamber 102, crushed and thermally vaporized. For example, the crushing and thermo-vaporization of the previously pyrolyzed hydrocarbon source rock sample can be performed simultaneously. During thermo-vaporization, the pyrolyzed hydrocarbon source rock sample 154 is placed in the thermo-vaporization chamber 152 and heated. A quantity of the pyrolyzed hydrocarbon source rock sample 154 used in thermo-vaporization can be a few tens of grams (for example, substantially 20 g). Fig. 4 is a plot 400 of an example of a thermo-vaporization temperature gradient according to which the pyrolyzed hydrocarbon source rock sample 154 can be thermally vaporized in the thermo-vaporization apparatus 150. For example, the temperature of the chamber 152 can be kept constant at substantially 300 °C time. Thermo-vaporization can purge any retained hydrocarbons in the rock sample.

At 210, hydrocarbons released by the pyrolyzed hydrocarbon source rock sample in response to the crushing and thermo-vaporization can be recovered. In some implementations, the chamber 152 can be continuously flushed with the inert carrier gas 158 (for example, helium or other inert carrier gas) using the hydrocarbons recovery apparatus. For example, the carrier gas 158 can be flowed into the thermo-vaporization chamber 152 through tubing 156 and out of the chamber 152 with the expelled hydrocarbons through tubing 160. As the hydrocarbon in the pyrolyzed hydrocarbon source rock sample 154 breaks down into gaseous form, the carrier gas 158 can carry the expelled hydrocarbons out of the crushing and thermo-vaporization chamber 152.

The combination of the hydrocarbons and the carrier gas 158 can be flowed to one or more cold traps (for example, the cold trap 166 or the cold trap 168 or both). In some implementations, each cold trap can be a fused silica column submerged in a container filled with liquid nitrogen or other low temperature (for example, as low as -100 °C). At the end of thermo-vaporization, the cold trap can be heated first to a temperature of about 60 °C and then to an elevated temperature of about 300 °C to purge the gaseous and liquid pyrolysis products, respectively. In this manner, the hydrocarbons trapped by the cold trap 168 can be released.

Similar to gas stream splitting during open system pyrolysis, the hydrocarbons recovery apparatus can be implemented to split the hydrocarbons flowing out of the thermo-vaporization chamber 152 into multiple gas streams. For example, the valve 162 can split the gas flowing from the crushing and thermo-vaporization chamber 152 into a first gas stream and a second gas stream. The first gas stream can be directed to a removable cold trap 168 and the second portion can be directed to the cold trap 166. The first gas stream flowed through the removable cold trap 166 can then be flowed to another instrumentation (not shown) to determine a composition of the gas stream. In some implementations, the first gas stream can be purged, for example, released to atmosphere. In some implementations, the gas stream exiting the crushing and thermo-vaporization chamber 152 can be split into multiple streams (that is, more than two streams). One of the streams can be flowed to the cold trap 168 and then to the instrumentation 172. Another stream can be flowed to the removable cold trap 166 and then to another instrumentation. A third stream can be purged, and so on. The splitting of the gas stream exiting the crushing and thermo-vaporization chamber 152 into multiple gas streams and the flow of the gas streams can be controlled, for example, using the valve 162.

In some implementations, the hydrocarbons carried by the carrier gas 158 out of the crushing and thermo-vaporization chamber 152 can be flowed to instrumentation 172 that can analyze a composition of the expelled hydrocarbons. For example, the instrumentation 174 can include a gas chromatograph (GC) or gas chromatography-Mass Spectrometer (GC-MS). The instrumentation 172 can be implemented to characterize the hydrocarbons expelled from the pyrolyzed hydrocarbon source rock sample 154. In some implementations, the instrumentation 172 can be implemented to quantify, for example, in micrograms of hydrocarbons per gram of hydrocarbon source rock sample.

In the example implementations described above, the hydrocarbon source rock sample 104 was placed in a pyrolysis chamber 102 during open system pyrolysis, and the pyrolyzed hydrocarbon source rock sample 154 was then placed in a separate and distinct sample crushing and thermo-vaporization chamber 152 during thermo-vaporization. Alternatively, the pyrolysis chamber 102 can be used as the sample crushing and thermo-vaporization chamber 152. Upon completion of the open system pyrolysis, the pyrolyzed hydrocarbon source rock sample 154 can be placed in (or can remain in) the pyrolysis chamber 152. Sample crushing and thermo-vaporization of the pyrolyzed hydrocarbon source rock sample can then be performed as described above.

In some implementations, at the end of each step (that is, each pyrolysis temperature stage, thermo-vaporization), the maturity of the residual kerogen can be determined using procedures such as geochemical techniques (for example, vitrinite reflectance measurements, rock eval pyrolysis, or other geochemical techniques). In this manner, after each step, a maturity level for the source rock which has generated, expelled or retained hydrocarbons can be characterized.

At 214, a hydrocarbon expulsion efficiency of the hydrocarbon rock can be determined. For example, the efficiency can be determined as a ratio of a sum of the first quantity of hydrocarbons (i.e., the hydrocarbons released during open system pyrolysis of the hydrocarbon source rock sample 104) to the second quantity of hydrocarbons (i.e., the hydrocarbons released during thermo-vaporization of the pyrolyzed hydrocarbon source rock sample 154). In some implementations, the hydrocarbon expulsion efficiency (HCEE) can be determined using Equation 1 shown below.

$$\text{HCEE} = (\text{HC}_{\text{Pyrolysis}}) \text{ x } 100 \text{ / } (\text{HC}_{\text{Pyrolysis}} + \text{HC}_{\text{CrushingThermo-Vaporization}}) \quad \text{(Equation 1)}$$

In Equation 1, $\text{HC}_{\text{Pyrolysis}}$ is the quantity (for example, in micrograms per gram of sample) of hydrocarbons released in response to open system pyrolysis of the hydrocarbon source rock sample 104. $\text{HC}_{\text{CrushingThermo-Vaporization}}$ is the quantity (for example, in micrograms per gram of sample) of hydrocarbons released in response to crushing and thermo-vaporization of the previously pyrolyzed hydrocarbon source rock sample 154.

At 216, a second hydrocarbon source rock sample can be obtained from the same hydrocarbon source rock from which the rock sample 104 was obtained. The rock sample can be ground to have grain sizes in the micrometer range (for example, less than substantially 250 $\mu$m). At 216, an open system pyrolysis can be performed on the powdered rock sample. A quantity of the powder size hydrocarbon source rock sample for the open system pyrolysis can be a few hundreds of milligrams to one gram (for example, substantially less than 1 gram). The open system pyrolysis on the second hydrocarbon rock sample can be performed using the open system pyrolysis apparatus 100 using techniques similar to those described above.

At 218, a quantity of hydrocarbons released in response to performing the open system pyrolysis of the powder hydrocarbon source rock sample can be determined using techniques similar to those described above.

In some implementations, as a verification of HCEE found by using Equation 1, the hydrocarbon expulsion efficiency (HCEE) can also be determined using Equation 2 shown below.

$$\text{HCEE} = (\text{HC}_{\text{Pyrolysis}}) \text{ x } 100 \text{ / } \text{HC}_{\text{PowderedSamplePyrolysis}} \quad \text{(Equation 2)}$$

In Equation 2, $\text{HC}_{\text{Pyrolysis}}$ is the quantity (for example, in micrograms per gram of sample) of hydrocarbons released in response to open system pyrolysis of the hydrocarbon rock sample 104, and $\text{HC}_{\text{PowderedSamplePyrolysis}}$ is the quantity (for example, in micrograms per gram of sample) of hydrocarbons released in response to open system pyrolysis of the powdered hydrocarbon source rock sample.

At 220, the hydrocarbon expulsion efficiency can be provided as an input to a geological model to study hydrocarbon expulsion from the hydrocarbon source rock thorough geological time.

In sum, the artificial maturation of hydrocarbon source rock samples using the techniques described in this specification can lead to better understanding of expulsion and retention of hydrocarbons in hydrocarbon source rocks in natural systems. Better understanding and quantification of expelled hydrocarbons versus retained hydrocarbons (with increasing maturation in laboratory experiments) can be quantified and converted into parameters. Such parameters can be provided as input to geological models, for example, basin modeling software, to calibrate the expulsion-retention of hydrocarbons that are generated during burial and maturation of hydrocarbon source rocks.

**Claims**

1. A method comprising:

   performing (202) an open system pyrolysis of a hydrocarbon source rock sample obtained from a natural system, the hydrocarbon source rock sample comprising hydrocarbon source rocks having an equivalent spherical diameter of substantially at least one centimeter;
   recovering (204) hydrocarbons released by the hydrocarbon source rock sample in response to the open system pyrolysis;

crushing the pyrolyzed hydrocarbon source rock;

after crushing the pyrolyzed hydrocarbon source rock, performing (208) a thermo-vaporization on the pyrolyzed hydrocarbon source rock sample on which the open system pyrolysis was performed;

recovering (210) hydrocarbons released by the pyrolyzed hydrocarbon source rock sample in response to the thermo-vaporization; and

determining (214) a hydrocarbon expulsion efficiency of hydrocarbon source rock in the natural system based on the recovered hydrocarbons released by the hydrocarbon source rock sample in response to the open system pyrolysis and based on the recovered hydrocarbons released by the pyrolyzed hydrocarbon source rock sample in response to thermo-vaporization.

2. The method of claim 1, wherein the natural system comprises a subsurface system comprising a hydrocarbon reservoir.

3. The method of claim 1, wherein:

the hydrocarbon source rock sample is first hydrocarbon source rock sample fragments obtained from a naturally occurring hydrocarbon source rock;

the crushing and thermo-vaporization is performed on the pyrolyzed hydrocarbon source rock sample on which the open system pyrolysis was performed; and

a hydrocarbon expulsion efficiency of hydrocarbon rock in a hydrocarbon reservoir is determined based on the recovered hydrocarbons released by the first hydrocarbon source rock sample in response to the open system pyrolysis and based on recovered hydrocarbons released by the pyrolyzed hydrocarbon source rock sample in response to the crushing and thermo-vaporization.

4. The method of claim 1, wherein determining the hydrocarbon expulsion efficiency comprises:

determining (206) a first quantity of hydrocarbons released in response to performing the open system pyrolysis; and

determining (212) a second quantity of hydrocarbons released in response to performing the crushing and thermo-vaporization, and optionally wherein determining the hydrocarbon expulsion efficiency further comprises determining a ratio of the first quantity of hydrocarbons to a sum of the first quantity of hydrocarbons and the second quantity of hydrocarbons.

5. The method of claim 1, wherein the open system pyrolysis of the hydrocarbon source rock sample is performed in a pyrolysis chamber (102), and wherein performing the open system pyrolysis of the hydrocarbon source rock sample comprises flowing a hydrocarbons transport through the pyrolysis chamber while performing the open system pyrolysis, the hydrocarbons transport configured to carry the hydrocarbons released by the hydrocarbon source rock sample.

6. The method of claim 5, wherein one of a) or b), wherein:

a) the hydrocarbons transport comprises an inert gas; or
b) a volume of the pyrolysis chamber is at least one liter.

7. The method of claim 1, wherein the thermo-vaporization on the pyrolyzed hydrocarbon source rock sample is performed in a thermo-vaporization chamber (152), and wherein performing the thermo-vaporization on the pyrolyzed hydrocarbon source rock sample comprises flowing an inert hydrocarbons transport through the thermo-vaporization chamber while performing the thermo-vaporization, the inert hydrocarbons transport configured to carry the hydrocarbons released from the pyrolyzed hydrocarbon source rock and which was retained within the sample during open system pyrolyis.

8. The method of claim 7, wherein the pyrolyzed hydrocarbon source rock sample is crushed in the thermo-vaporization chamber prior to performing the thermo-vaporization, and optionally wherein the open system pyrolysis of the hydrocarbon source rock sample, crushing the pyrolyzed hydrocarbon source rock sample, and thermo-vaporization of the pyrolyzed hydrocarbon source rock sample are performed in the thermo-vaporization chamber.

9. The method of claim 1, wherein a quantity of the hydrocarbon source rock sample used in the open system pyrolysis is substantially at least 100 g.

10. The method of claim 1, wherein recovering hydrocarbons released by the hydrocarbon source rock sample in response to the open system pyrolysis comprises flowing the hydrocarbons released by the hydrocarbon source rock sample to a cold trap (116, 118) comprising a fused silica column submerged in liquid nitrogen, and optionally wherein the method further comprises

maintaining the cold trap at a temperature of substantially -100 °C until an end of the open system pyrolysis, wherein the cold trap traps at least a portion of the hydrocarbons released by the hydrocarbon source rock sample, and
heating the cold trap after the end of the open system pyrolysis to release the trapped portion of the hydrocarbons.

11. The method of claim 1, further comprising flowing the recovered hydrocarbons released by the hydrocarbon source rock sample in response to the open system pyrolysis to instrumentation configured to analyze composition of the recovered hydrocarbons, wherein for example, the instrumentation comprises a gas chromatograph.

12. The method of claim 11, further comprising:

splitting the recovered hydrocarbons released by the hydrocarbon source rock sample into a plurality of hydrocarbon gas streams;
flowing a first stream to a first instrumentation configured to analyze a composition of the first stream; and
flowing a second stream to a second instrumentation configured to analyze a composition of the second stream, and optionally wherein the method further comprises releasing a third stream to atmosphere.

13. The method of claim 1, wherein performing the open system pyrolysis of the hydrocarbon source rock sample comprises heating the hydrocarbon source rock sample to one or more of a plurality of temperatures up to 650 °C, and optionally wherein heating the hydrocarbon source rock sample to one or more of the plurality of temperatures comprises heating the hydrocarbon source rock sample at different heating rates, wherein a heating rate is a time rate of temperature increase from a lower temperature to a higher temperature.

14. The method of claim 1, wherein performing the thermo-vaporization on the pyrolyzed hydrocarbon source rock sample comprises heating the pyrolyzed hydrocarbon source rock sample at constant temperature of substantially 300 °C.

15. The method of claim 1, further comprising providing the hydrocarbon expulsion efficiency as an input to a computer-generated geological model to study hydrocarbon expulsion efficiency from a hydrocarbon source rock through a geological history of the hydrocarbon source rock.

16. A system for performing the method of any preceding claim, the system comprising:

an open system pyrolysis apparatus (100) configured to pyrolyze fragments of a hydrocarbon source rock sample, the fragments having an equivalent spherical diameter of substantially at least one centimeter;
a crushing and thermo-vaporization apparatus (150) configured to crush and simultaneously thermally vaporize released hydrocarbons from previously pyrolyzed hydrocarbon source rock sample;
a hydrocarbon transport apparatus (108, 158) configured to recover hydrocarbons released by the hydrocarbon source rock sample in response to the open system pyrolysis and hydrocarbons released by the previously pyrolyzed hydrocarbon source rock sample in response to the crushing and thermo-vaporization; and
instrumentation (122, 172) configured to receive the recovered hydrocarbons released by the hydrocarbon source rock sample in response to the open system pyrolysis and the hydrocarbons released by the pyrolyzed hydrocarbon source rock sample in response to the crushing and thermo-vaporization.

**Patentansprüche**

1. Verfahren, umfassend:

Durchführen (202) einer Offensystem-Pyrolyse einer Kohlenwasserstoffquellen-Gesteinsprobe, die aus einem natürlichen System erhalten wird, wobei die Kohlenwasserstoffquellen-Gesteinsprobe Kohlenwasserstoffquellen-Gesteine mit einem äquivalenten sphärischen Durchmesser von im Wesentlichen mindestens einem Zentimeter umfasst;

Gewinnung (204) von Kohlenwasserstoffen, die von der Kohlenwasserstoffquellen-Gesteinsprobe als Reaktion auf die Offensystem-Pyrolyse freigesetzt werden;

Zerkleinern des pyrolysierten Kohlenwasserstoffquellen-Gesteins;

nach dem Zerkleinern des pyrolysierten Kohlenwasserstoffquellen-Gesteins, Durchführen (208) einer thermischen Verdampfung auf der pyrolysierten Kohlenwasserstoffquellen-Gesteinsprobe, auf der die Offensystem-Pyrolyse durchgeführt wurde;

Gewinnung (210) von Kohlenwasserstoffen, die von der Kohlenwasserstoffquellen-Gesteinsprobe als Reaktion auf die thermische Verdampfung freigesetzt werden; und

Bestimmen (214) einer Kohlenwasserstoff-Expulsionseffizienz des Kohlenwasserstoffquellen-Gesteins in dem natürlichen System basierend auf den gewonnenen Kohlenwasserstoffen, die von der Kohlenwasserstoffquellen-Gesteinsprobe als Reaktion auf die Offensystem-Pyrolyse freigesetzt werden, und basierend auf den gewonnenen Kohlenwasserstoffen, die von der pyrolysierten Kohlenwasserstoffquellen-Gesteinsprobe als Reaktion auf eine thermische Verdampfung freigesetzt werden.

2. Verfahren nach Anspruch 1, wobei das natürliche System ein unterirdisches System umfasst, welches einen Kohlenwasserstoffspeicher umfasst.

3. Verfahren nach Anspruch 1, wobei:

die Kohlenwasserstoffquellen-Gesteinsprobe erste Fragmente einer Kohlenwasserstoffquellen-Gesteinsprobe sind, die aus einem natürlich vorkommenden Kohlenwasserstoffquellen-Gestein erhalten werden;

das Zerkleinern und die thermische Verdampfung auf der pyrolysierten Kohlenwasserstoffquellen-Gesteinsprobe durchgeführt werden, auf der die Offensystem-Pyrolyse durchgeführt wurde; und

eine Kohlenwasserstoff-Expulsionseffizienz des Kohlenwasserstoffquellen-Gesteins in einem Kohlenwasserstoffspeicher basierend auf den gewonnenen Kohlenwasserstoffen, die von der ersten Kohlenwasserstoffquellen-Gesteinsprobe als Reaktion auf die Offensystem-Pyrolyse freigesetzt werden, und basierend auf den gewonnenen Kohlenwasserstoffen, die von der pyrolysierten Kohlenwasserstoffquellen-Gesteinsprobe als Reaktion auf das Zerkleinern und die thermische Verdampfung freigesetzt werden, bestimmt wird.

4. Verfahren nach Anspruch 1, wobei das Bestimmen der Kohlenwasserstoff-Expulsionseffizienz umfasst:

Bestimmen (206) einer ersten Menge von Kohlenwasserstoffen, die freigesetzt wird, wenn die Offensystem-Pyrolyse durchgeführt wird; und

Bestimmen (212) einer zweiten Menge von Kohlenwasserstoffen, die freigesetzt wird, wenn das Zerkleinern und die thermische Verdampfung durchgeführt werden, und wobei das Bestimmen der Kohlenwasserstoff-Expulsionseffizienz optional ferner ein Bestimmen eines Verhältnisses der ersten Menge von Kohlenwasserstoffen zu einer Summe der ersten Menge von Kohlenwasserstoffen und der zweiten Menge von Kohlenwasserstoffen umfasst.

5. Verfahren nach Anspruch 1, wobei die Offensystem-Pyrolyse der Kohlenwasserstoffquellen-Gesteinsprobe in einer Pyrolyse-Kammer (102) durchgeführt wird und wobei das Durchführen der Offensystem-Pyrolyse der Kohlenwasserstoffquellen-Gesteinsprobe ein Leiten eines Kohlenwasserstofftransports durch die Pyrolyse-Kammer umfasst, während die Offensystem-Pyrolyse durchgeführt wird, wobei der Kohlenwasserstofftransport eingerichtet ist, um die Kohlenwasserstoffe zu tragen, die von der Kohlenwasserstoffquellen-Gesteinsprobe freigesetzt werden.

6. Verfahren nach Anspruch 5, wobei entweder a) oder b), wobei:

a) der Kohlenwasserstofftransport ein Inertgas umfasst; oder
b) ein Volumen der Pyrolyse-Kammer mindestens einen Liter beträgt.

7. Verfahren nach Anspruch 1, wobei die thermische Verdampfung auf der pyrolysierten Kohlenwasserstoffquellen-Gesteinsprobe in einer Thermoverdampfungskammer (152) durchgeführt wird und wobei das Durchführen der thermischen Verdampfung auf der pyrolysierten Kohlenwasserstoffquellen-Gesteinsprobe ein Leiten eines Transports inerter Kohlenwasserstoffe durch die Thermoverdampfungskammer umfasst, während die thermischen Verdampfung durchgeführt wird, wobei der Transport inerter Kohlenwasserstoffe eingerichtet ist, um die Kohlenwasserstoffe zu tragen, die von dem pyrolysierten Kohlenwasserstoffquellen-Gestein freigesetzt werden und die in der Probe während der Offensystem-Pyrolyse enthalten waren.

8. Verfahren nach Anspruch 7, wobei die pyrolysierte Kohlenwasserstoffquellen-Gesteinsprobe in der Thermoverdampfungskammer zerkleinert wird, bevor die thermische Verdampfung durchgeführt wird, und wobei die Offensystem-Pyrolyse der Kohlenwasserstoffquellen-Gesteinsprobe, das Zerkleinern der pyrolysierten Kohlenwasserstoffquellen-Gesteinsprobe und die thermische Verdampfung der pyrolysierten Kohlenwasserstoffquellen-Gesteinsprobe optional in der Thermoverdampfungskammer durchgeführt werden.

9. Verfahren nach Anspruch 1, wobei eine Menge der Kohlenwasserstoffquellen-Gesteinsprobe, die in der Offensystem-Pyrolyse verwendet wird, im Wesentlichen mindestens 100 g beträgt.

10. Verfahren nach Anspruch 1, wobei die Gewinnung von Kohlenwasserstoffen, die von der Kohlenwasserstoffquellen-Gesteinsprobe als Reaktion auf die Offensystem-Pyrolyse freigesetzt werden, ein Leiten der Kohlenwasserstoffe, die von der Kohlenwasserstoffquellen-Gesteinsprobe freigesetzt werden, zu einer Kühlfalle (116, 118) umfasst, welche eine Quarzglas-Säule umfasst, die in flüssigen Stickstoff eingetaucht ist, und wobei das Verfahren optional ferner umfasst:

Halten der Kühlfalle auf einer Temperatur von im Wesentlichen -100 °C bis zu einem Abschluss der Offensystem-Pyrolyse, wobei die Kühlfalle mindestens einen Teil der Kohlenwasserstoffe, die von der Kohlenwasserstoffquellen-Gesteinsprobe freigesetzt werden, abscheidet, und
Erwärmen der Kühlfalle nach dem Abschluss der Offensystem-Pyrolyse, um den abgeschiedenen Teil der Kohlenwasserstoffe freizusetzen.

11. Verfahren nach Anspruch 1, ferner umfassend Leiten der gewonnenen Kohlenwasserstoffe, die von der Kohlenwasserstoffquellen-Gesteinsprobe als Reaktion auf die Offensystem-Pyrolyse freigesetzt werden, zu Ausrüstungen, die eingerichtet sind, um eine Zusammensetzung der gewonnenen Kohlenwasserstoffe zu analysieren, wobei die Ausrüstungen zum Beispiel einen Gas-Chromatograph umfassen.

12. Verfahren nach Anspruch 11, ferner umfassend:

Aufteilen der gewonnenen Kohlenwasserstoffe, die von der Kohlenwasserstoffquellen-Gesteinsprobe freigesetzt werden, in mehrere Kohlenwasserstoff-Gasströme;
Leiten eines ersten Stroms zu einer ersten Ausrüstung, die eingerichtet ist, um eine Zusammensetzung des ersten Stroms zu analysieren; und
Leiten eines zweiten Stroms zu einer zweiten Ausrüstung, die eingerichtet ist, um eine Zusammensetzung des zweiten Stroms zu analysieren, und wobei das Verfahren optional ferner ein Freisetzen eines dritten Stroms in die Atmosphäre umfasst.

13. Verfahren nach Anspruch 1, wobei das Durchführen der Offensystem-Pyrolyse der Kohlenwasserstoffquellen-Gesteinsprobe ein Erwärmen der Kohlenwasserstoffquellen-Gesteinsprobe auf eine oder mehrere von mehreren Temperaturen von bis zu 650 °C umfasst, und wobei das Erwärmen der Kohlenwasserstoffquellen-Gesteinsprobe auf eine oder mehrere der mehreren Temperaturen optional ein Erwärmen der Kohlenwasserstoffquellen-Gesteinsprobe mit unterschiedlichen Erwärmungsraten umfasst, wobei eine Erwärmungsrate eine Zeitrate eines Temperaturanstiegs von einer niedrigeren Temperatur auf eine höhere Temperatur ist.

14. Verfahren nach Anspruch 1, wobei das Durchführen der thermischen Verdampfung auf der pyrolysierten Kohlenwasserstoffquellen-Gesteinsprobe ein Erwärmen der pyrolysierten Kohlenwasserstoffquellen-Gesteinsprobe bei einer konstanten Temperatur von im Wesentlichen 300 °C umfasst.

15. Verfahren nach Anspruch 1, ferner umfassend Bereitstellen der Kohlenwasserstoff-Expulsionseffizienz als Eingabe in ein computererzeugtes geologisches Modell, um die Kohlenwasserstoff-Expulsionseffizienz von einem Kohlenwasserstoffquellen-Gestein im Verlauf der geologischen Geschichte des Kohlenwasserstoffquellen-Gesteins zu untersuchen.

16. System zum Durchführen des Verfahrens nach einem vorhergehenden Anspruch, wobei das System umfasst:

eine Offensystem-Pyrolyse-Vorrichtung (100), die eingerichtet ist, um Fragmente einer Kohlenwasserstoffquellen-Gesteinsprobe zu pyrolysieren, wobei die Fragmente einen äquivalenten sphärischen Durchmesser von im Wesentlichen mindestens einem Zentimeter aufweisen;
eine Zerkleinerungs- und Thermoverdampfungsvorrichtung (150), die eingerichtet ist, um die freigesetzten Kohlenwasserstoffe von einer zuvor pyrolysierten Kohlenwasserstoffquellen-Gesteinsprobe zu zerkleinern und gleichzeitig thermisch verdampfen zu lassen;

eine Kohlenwasserstoff-Transportvorrichtung (108, 158), die eingerichtet ist, um Kohlenwasserstoffe, die von der Kohlenwasserstoffquellen-Gesteinsprobe als Reaktion auf die Offensystem-Pyrolyse freigesetzt werden, und Kohlenwasserstoffe, die von der zuvor pyrolysierten Kohlenwasserstoffquellen-Gesteinsprobe als Reaktion auf das Zerkleinern und die thermische Verdampfung freigesetzt werden, zu gewinnen; und

Ausrüstungen (122, 172), die eingerichtet sind, um die gewonnenen Kohlenwasserstoffe, die von der Kohlenwasserstoffquellen-Gesteinsprobe als Reaktion auf die Offensystem-Pyrolyse freigesetzt werden, und die Kohlenwasserstoffe, die von der pyrolysierten Kohlenwasserstoffquellen-Gesteinsprobe als Reaktion auf das Zerkleinern und die thermische Verdampfung freigesetzt werden, aufzunehmen.

## Revendications

1. Procédé comprenant :

   - l'exécution (202) d'une pyrolyse à système ouvert d'un échantillon de roche mère d'hydrocarbures obtenu à partir d'un système naturel, l'échantillon de roche mère d'hydrocarbures comprenant des roches mères d'hydrocarbures présentant un diamètre sphérique équivalent mesurant substantiellement au moins un centimètre ;
   - la récupération (204) d'hydrocarbures libérés par l'échantillon de roche mère d'hydrocarbures en réponse à la pyrolyse à système ouvert ;
   - le concassage de la roche mère d'hydrocarbures pyrolysée ;
   - l'exécution (208), après le concassage de la roche mère d'hydrocarbures pyrolysée, d'une vaporisation thermique sur l'échantillon de roche mère d'hydrocarbures pyrolysé sur lequel la pyrolyse à système ouvert a été réalisée ;
   - la récupération (210) d'hydrocarbures libérés par l'échantillon de roche mère d'hydrocarbures pyrolysé en réponse à la vaporisation thermique ; et
   - la détermination (214) d'une efficacité d'expulsion d'hydrocarbures de la roche mère d'hydrocarbures dans le système naturel, sur la base des hydrocarbures récupérés libérés par l'échantillon de roche mère d'hydrocarbures en réponse à la pyrolyse à système ouvert et sur la base des hydrocarbures récupérés libérés par l'échantillon de roche mère d'hydrocarbures pyrolysé en réponse à la vaporisation thermique.

2. Procédé selon la revendication 1, dans lequel le système naturel comprend un système souterrain comprenant un réservoir d'hydrocarbures.

3. Procédé selon la revendication 1, dans lequel :

   - l'échantillon de roche mère d'hydrocarbures consiste en des premiers fragments d'échantillon de roche mère d'hydrocarbures, obtenus à partir d'une roche mère d'hydrocarbures naturelle ;
   - le concassage et la vaporisation thermique sont réalisés sur l'échantillon de roche mère d'hydrocarbures pyrolysé sur lequel la pyrolyse à système ouvert a été réalisée ; et
   - une efficacité d'expulsion d'hydrocarbures de la roche d'hydrocarbures dans un réservoir d'hydrocarbures est déterminée sur la base des hydrocarbures récupérés libérés par le premier échantillon de roche mère d'hydrocarbures en réponse à la pyrolyse à système ouvert et sur la base d'hydrocarbures récupérés libérés par l'échantillon de roche mère d'hydrocarbures pyrolysé en réponse au concassage et à la vaporisation thermique.

4. Procédé selon la revendication 1, dans lequel la détermination de l'efficacité d'expulsion d'hydrocarbures comprend :

   - la détermination (206) d'une première quantité d'hydrocarbures libérée en réponse à la réalisation de la pyrolyse à système ouvert ; et
   - la détermination (212) d'une deuxième quantité d'hydrocarbures libérée en réponse à la réalisation du concassage et de la vaporisation thermique, et dans lequel la détermination de l'efficacité d'expulsion d'hydrocarbures comprend en outre facultativement la détermination d'un rapport de la première quantité d'hydrocarbures à une somme de la première quantité d'hydrocarbures et de la deuxième quantité d'hydrocarbures.

5. Procédé selon la revendication 1, dans lequel la pyrolyse à système ouvert de l'échantillon de roche mère d'hydrocarbures est réalisée dans une chambre de pyrolyse (102), et dans lequel la réalisation de la pyrolyse à système ouvert de l'échantillon de roche mère d'hydrocarbures comprend l'écoulement d'un transport d'hydrocarbures à travers la chambre de pyrolyse pendant la réalisation de la pyrolyse à système ouvert, le transport d'hydrocarbures étant configuré pour transporter les hydrocarbures libérés par l'échantillon de roche mère d'hydrocarbures.

6. Procédé selon la revendication 5, dans lequel l'un parmi a) ou b), dans lequel :

   a) le transport d'hydrocarbures comprend un gaz inerte ; ou
   b) un volume de la chambre de pyrolyse est d'au moins un litre.

7. Procédé selon la revendication 1, dans lequel la vaporisation thermique sur l'échantillon de roche mère d'hydrocarbures pyrolysé est réalisée dans une chambre de vaporisation thermique (152),
   et dans lequel la réalisation de la vaporisation thermique sur l'échantillon de roche mère d'hydrocarbures pyrolysé comprend l'écoulement d'un transport d'hydrocarbures inertes à travers la chambre de vaporisation thermique pendant la réalisation de la vaporisation thermique, le transport d'hydrocarbures inertes étant configuré pour transporter les hydrocarbures libérés à partir de la roche mère d'hydrocarbures pyrolysée et ayant été retenus dans l'échantillon pendant la pyrolyse à système ouvert.

8. Procédé selon la revendication 7, dans lequel l'échantillon de roche mère d'hydrocarbures pyrolysé est concassé dans la chambre de vaporisation thermique avant la réalisation de la vaporisation thermique, et dans lequel la pyrolyse à système ouvert de l'échantillon de roche mère d'hydrocarbures, le concassage de l'échantillon de roche mère d'hydrocarbures pyrolysé, et la vaporisation thermique de l'échantillon de roche mère d'hydrocarbures pyrolysé sont facultativement réalisés dans la chambre de vaporisation thermique.

9. Procédé selon la revendication 1, dans lequel une quantité de l'échantillon de roche mère d'hydrocarbures utilisée dans la pyrolyse à système ouvert est substantiellement d'au moins 100 g.

10. Procédé selon la revendication 1, dans lequel la récupération d'hydrocarbures libérés par l'échantillon de roche mère d'hydrocarbures en réponse à la pyrolyse à système ouvert comprend l'écoulement des hydrocarbures libérés par l'échantillon de roche mère d'hydrocarbures vers un piège froid (116, 118) comprenant une colonne en silice fondue immergée dans de l'azote liquide, et dans lequel le procédé comprend facultativement également

   - le maintien du piège froid à une température substantiellement égale à -100°C jusqu'à une fin de la pyrolyse à système ouvert, le piège froid piégeant au moins une partie des hydrocarbures libérés par l'échantillon de roche mère d'hydrocarbures, et
   - le chauffage du piège froid après la fin de la pyrolyse à système ouvert pour libérer la partie piégée des hydrocarbures.

11. Procédé selon la revendication 1, comprenant en outre l'écoulement des hydrocarbures récupérés libérés par l'échantillon de roche mère d'hydrocarbures en réponse à la pyrolyse à système ouvert vers une instrumentation configurée pour analyser la composition des hydrocarbures récupérés, l'instrumentation comprenant par exemple un chromatographe en phase gazeuse.

12. Procédé selon la revendication 11, comprenant en outre :

   - la division des hydrocarbures récupérés libérés par l'échantillon de roche mère d'hydrocarbures en une pluralité de flux de gaz d'hydrocarbures ;
   - l'écoulement d'un premier flux vers une première instrumentation configurée pour analyser une composition du premier flux ; et
   - l'écoulement d'un deuxième flux vers une deuxième instrumentation configurée pour analyser une composition du deuxième flux, et le procédé comprenant facultativement également la libération d'un troisième flux vers l'atmosphère.

13. Procédé selon la revendication 1, dans lequel la réalisation de la pyrolyse à système ouvert de l'échantillon de roche mère d'hydrocarbures comprend le chauffage de l'échantillon de roche mère d'hydrocarbures à une ou plusieurs parmi une pluralité de températures allant jusqu'à 650°C, et dans lequel le chauffage de l'échantillon de roche mère d'hydrocarbures à une ou plusieurs parmi une pluralité de températures comprend facultativement le chauffage de l'échantillon de roche mère d'hydrocarbures à différentes vitesses de chauffage, une vitesse de chauffage étant un taux de temps d'augmentation de température à partir d'une température plus basse jusqu'à une température plus élevée.

**14.** Procédé selon la revendication 1, dans lequel la réalisation de la vaporisation thermique sur l'échantillon de roche mère d'hydrocarbures pyrolysé comprend le chauffage de l'échantillon de roche mère d'hydrocarbures pyrolysé à une température constante de substantiellement 300°C.

**15.** Procédé selon la revendication 1, comprenant en outre l'apport de l'efficacité d'expulsion d'hydrocarbures comme entrée dans un modèle géologique généré par ordinateur, pour l'étude de l'efficacité d'expulsion d'hydrocarbures à partir d'une roche mère d'hydrocarbures à travers une histoire géologique de la roche mère d'hydrocarbures.

**16.** Système pour la mise en œuvre du procédé selon l'une quelconque des revendications précédentes, le système comprenant :

- un appareil de pyrolyse à système ouvert (100) configuré pour pyrolyser des fragments d'un échantillon de roche mère d'hydrocarbures, les fragments présentant un diamètre sphérique équivalent mesurant substantiellement au moins un centimètre ;
- un appareil de concassage et de vaporisation thermique (150) configuré pour concasser et simultanément vaporiser thermiquement des hydrocarbures libérés provenant de l'échantillon de roche mère d'hydrocarbures pyrolysé précédemment ;
- un appareil de transport d'hydrocarbures (108, 158) configuré pour récupérer des hydrocarbures libérés par l'échantillon de roche mère d'hydrocarbures en réponse à la pyrolyse à système ouvert et des hydrocarbures libérés par l'échantillon de roche mère d'hydrocarbures précédemment pyrolysé en réponse au concassage et à la vaporisation thermique ; et
- une instrumentation (122, 172) configurée pour recevoir les hydrocarbures récupérés libérés par l'échantillon de roche mère d'hydrocarbures en réponse à la pyrolyse à système ouvert et les hydrocarbures libérés par l'échantillon de roche mère d'hydrocarbures précédemment pyrolysé en réponse au concassage et à la vaporisation thermique.

FIG. 1A

FIG. 1B

**200**

202 — PERFORMING AN OPEN SYSTEM PYROLYSIS ON A HYDROCARBON ROCK SAMPLE COMPRISING FRAGMENTS OF HYDROCARBON ROCKS HAVING AN EQUIVALENT SPHERICAL DIAMETER OF SUBSTANTIALLY AT LEAST ONE CENTIMETER

204 — RECOVERING HYDROCARBONS RELEASED BY THE ROCK SAMPLE IN RESPONSE TO THE OPEN SYSTEM PYROLYSIS

206 — DETERMINING A FIRST QUANTITY OF HYDROCARBONS RELEASED IN RESPONSE TO PERFORMING THE OPEN SYSTEM PYROLYSIS

208 — CRUSHING AND PERFORMING THERMO-VAPORIZATION ON THE PYROLYZED HYDROCARBON ROCK SAMPLE

210 — RECOVERING HYDROCARBON GAS RELEASED BY THE CRUSHED AND PYROLYZED HYDROCARBON ROCK SAMPLE IN RESPONSE TO THE THERMO-VAPORIZATION

212 — DETERMINING A SECOND QUANTITY OF HYDROCARBONS RELEASED IN RESPONSE TO PERFORMING THE THERMO-VAPORIZATION OF THE CRUSHED SAMPLE

214 — DETERMINING A HYDROCARBON EXPULSION EFFICIENCY OF THE HYDROCARBON ROCK AS A RATIO OF A SUM OF THE FIRST QUANTITY OF HYDROCARBONS TO A SUM OF THE FIRST QUANTITY OF HYDROCARBONS AND THE SECOND QUANTITY OF HYDROCARBONS

216 — PYROLYZING A POWDERED SAMPLE OF THE HYDROCARBON SOURCE ROCK SAMPLE HAVING GRAIN SIZE < 250 MICRONS

218 — DETERMINING A THIRD QUANTITY OF HYDROCARBONS RELEASED IN RESPONSE TO PERFORMING OPEN SYSTEM PYROLYSIS ON A POWDERED SAMPLE

220 — PROVIDING THE HYDROCARBON EXPULSION EFFICIENCY AS AN INPUT TO A GEOLOGICAL MODEL TO STUDY HYDROCARBON EXPULSION FROM THE HYDROCARBON SOURCE ROCK

## FIG. 2

300

TEMPERATURE °C

600

500

400

300

VII

VI

V

IV

III

II

I

TIME (MINUTES)

FIG. 3

400

TEMPERATURE °C    300

TIME (MINUTES)

FIG. 4

FIG. 5A

FIG. 5B

MATURITY (% Ro)

PYRO T (°C)

500

550

C1

C2-C4

C5+

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 71782515

- GB 2161269 A

**Non-patent literature cited in the description**

- **CHRIS CORNFORD.** Risking Petroleum Systems. *Proc. 5th Int. Con. Exhib. Petrol. Geochem. Explor. Afro-Asian Region,* 01 January 2000

- **ESEME et al.** Experimental investigation of the compositional variation of petroleum during primary migration. *Org. Geochem,* 2007, vol. 38 (8), 1373-1397